# FASCICULE DE BREVET EUROPEEN

(11) **EP 2 793 684 B1**
(45) Date de publication et mention de la délivrance du brevet: **10.06.2020**
(21) Numéro de dépôt: 12813930.0
(22) Date de dépôt: 14.12.2012
(51) Int. Cl.: A61B 3/103

(54) **DISPOSITIF DE MULTIPLEXAGE BINOCULAIRE COMBINÉ À UN INSTRUMENT OPHTALMOLOGIQUE ET PROCÉDÉ DE DÉTERMINATION D'AU MOINS UN PARAMÈTRE DE VISION BINOCULAIRE D'UN SUJET**
BINOKULARES MULTIPLEXVORRICHTUNG MIT OPHTHALMISCHES INSTRUMENT UND VERFAHREN ZUR MESSUNG VON ZUMINDEST EINEN BINOKULAREN VISIONSPARAMETER
BINOCULAR MULTIPLEXING DEVICE COMBINED WITH AN OPHTHALMIC INSTRUMENT AND METHOD FOR DETERMINING AT LEAST ONE BINOCULAR VISION PARAMETER OF A SUBJECT

(30) Priorité: 22.12.2011 FR 1104037
(43) Date de publication de la demande: 29.10.2014
(73) Titulaire: Essilor International, 94220 Charenton-le-Pont (FR)
(72) Inventeur: BARANTON, Konogan, F-94220 Charenton-Le-Pont (FR); ESCALIER, Guilhem, F-94220 Charenton-Le-Pont (FR); ROUSSEAU, Benjamin, F-94220 Charenton-Le-Pont (FR)
(74) Mandataire: Jacobacci Coralis Harle
(86) Numéro de dépôt international: PCT/FR2012/052946
(87) Numéro de publication internationale: WO 2013/093308

(56) Documents cités:
- JP-A- 7 088 081
- JP-A- H0 775 623
- US-A- 5 777 718
- US-A- 5 872 614
- US-B1- 6 309 068

## Description

### DOMAINE TECHNIQUE AUQUEL SE RAPPORTE L'INVENTION

La présente invention concerne de manière générale le domaine des dispositifs, instruments et procédés d'optométrie binoculaires. Plus particulièrement, l'invention concerne un appareil d'optométrie basé sur la mesure de la réflexion et/ou de la réfraction d'un faisceau d'éclairage par chaque œil. Les mesures d'optométrie visent à déterminer les valeurs de la prescription d'une paire de lentilles de lunettes de compensation visuelle. Ces mesures binoculaires sont destinées à être exploitées pour la conception optique et la fabrication des faces de réfraction de verres de lunettes de compensation en vision de loin, de près (y compris lunettes de type readers, lunettes pré-montées), ayant des verres passifs ou de puissances optiques variables par commande électronique.

### ARRIERE-PLAN TECHNOLOGIQUE

La plupart des instruments d'optométrie dans le commerce sont monoculaires. Un premier type d'appareil monoculaire repose sur un dispositif monoculaire comprenant un stimulus et un système de mesure monoculaire. Le dispositif monoculaire est placé devant un œil pour effectuer une première mesure puis déplacé devant l'autre œil pour effectuer une deuxième mesure. L'inconvénient d'une double mesure monoculaire est d'être effectuée dans des conditions éloignées de la vision naturelle du sujet, qui est binoculaire. Notamment en vision de près, la convergence prismatique change l'axe de visée des yeux. Des contraintes physiologiques telles que des efforts musculaires apparaissent qui peuvent modifier la réfraction des yeux ou la pression de la paupière sur la cornée. Selon les sujets, une mesure en vision de près dans l'axe de visée d'un stimulus binoculaire peut être différente d'une mesure en vision monoculaire suivant un axe de visée droit devant. De plus, dans un système monoculaire, le changement d'œil nécessite un déplacement du stimulus et du système de mesure. Ainsi, le sujet voit le stimulus avec le premier œil mesuré, puis lors du changement, les deux yeux ne sont plus stimulés jusqu'à ce que l'appareil arrive devant l'autre œil. Ce déplacement impose aux yeux du sujet un changement d'intensité lumineuse, libérant temporairement le sujet du stimulus et pouvant provoquer une dilatation ou contraction pupillaire, comme un clignement des yeux. Ce laps de temps entre deux mesures monoculaires peut modifier les repères et le référentiel, faussant ainsi les mesures.

Il existe des solutions où le stimulus est binoculaire et la mesure monoculaire. Le système de mesure monoculaire nécessite un déplacement mécanique entre chaque œil. Bien que le changement d'alignement du système de mesure monoculaire soit invisible pour le sujet, le bruit ou les vibrations peuvent le détourner du stimulus, modifiant son accommodation, la taille et/ou la position de sa pupille. De plus, la mesure binoculaire reste basée sur deux mesures monoculaires séquentielles. Or, plus le laps de temps entre deux mesures monoculaires est court et plus la différence d'accommodation entre les deux yeux est faible, limitant ainsi les erreurs de mesure induites par les fluctuations d'accommodation.

Il existe aussi des instruments de mesure binoculaire comportant deux appareils de mesure monoculaires alignés respectivement sur chacun des deux yeux, chaque appareil de mesure monoculaire comportant un stimulus propre. Dans les instruments d'optométrie binoculaires, les deux stimuli sont généralement basés sur les mêmes représentations visuelles ou les mêmes motifs, mais les deux stimuli sont physiquement différents. Toutefois, les deux stimuli ne sont pas parfaitement identiques pour les deux yeux, en termes d'inclinaison ou d'orientation relative de l'un par rapport à l'autre par exemple. L'inconvénient d'une telle solution est la difficulté pour le sujet de fusionner les deux stimuli. En effet, la périphérie du stimulus contribue beaucoup au succès de la fusion par le sujet, en plus du centre du stimulus visé. Les personnes ayant des difficultés à fusionner deux images sont extrêmement sensibles aux différences entre leur vision droite/gauche.

Pour favoriser la fusion des stimuli, il est donc préférable de partir du même élément physique de stimulation. Les fluctuations d'accommodation sont ainsi réduites ce qui facilite la vision binoculaire pour les personnes ayant du mal à fusionner deux images distinctes.

Il existe aussi des systèmes qui intègrent un système de vision binoculaire d'un stimulus commun avec un moyen de mesure double pour caractériser les deux yeux en même temps. Dans ce cas, le capteur de mesure reçoit dans son champ de mesure les signaux provenant des deux yeux. Cette solution impose un capteur de mesure plus large, plus complexe et onéreux. De plus, cette solution limite la dynamique et la résolution de la mesure. Un capteur de mesure à champ large est spécifique et n'est pas dans le standard des appareils couramment utilisés.

Le document JP H07 75623 A concerne un réfractomètre ophtalmique comprenant un miroir pour commuter le faisceau d'éclairage et le faisceau réfléchi entre l'œil gauche et l'œil droit.

Il n'est généralement pas possible d'utiliser un stimulus et un moyen de mesure provenant d'un autre appareil (autorefractomètre, aberromètre ...) pour créer une vision binoculaire du stimulus et de la mesure sélectionnée.

### OBJET DE L'INVENTION

On cherche à effectuer des mesures ophtalmologiques binoculaires. En particulier, on cherche à effectuer des mesures de réfraction et/ou réflexion par les deux yeux simultanément. A titre complémentaire, on cherche à effectuer des mesures binoculaires d'astigmatisme ou d'aberration d'ordre supérieur.

Un des buts de l'invention est de fournir un dispositif et une méthode d'optométrie pour effectuer une mesure d'un paramètre ophtalmologique binoculaire de réfraction et/ou de réflexion par l'oeil d'un sujet en conditions prédéterminées de vision binoculaire.

L'invention vise à proposer un dispositif d'optométrie pour mesurer au moins un paramètre de vision binoculaire en position naturelle de regard du sujet.

Afin de remédier aux inconvénients précités de l'état de la technique, la présente invention propose un dispositif de multiplexage binoculaire combiné à un instrument ophtalmologique à plusieurs voies de mesure objective d'au moins un paramètre de vision d'un sujet, selon la revendication 1.

D'autres caractéristiques non limitatives et avantageuses du dispositif de multiplexage binoculaire conforme à l'invention sont les suivantes :
- lesdits premiers moyens de séparation optique comprennent une lame séparatrice ou un cube séparateur de faisceau ;
- ledit commutateur optique comprend une lame dichroïque, un miroir orientable ou escamotable ;
- lesdits seconds moyens de séparation optique comprennent une lame séparatrice ou un cube séparateur.

Avantageusement, lesdits premiers moyens de combinaison optique comprennent une lame dichroïque apte à superposer un faisceau de stimulation oculaire droit dans le domaine visible et un faisceau d'éclairage monoculaire droit dans le domaine infrarouge, et/ou respectivement lesdits seconds moyens de combinaison optique comprennent une lame dichroïque apte à superposer un faisceau de stimulation oculaire gauche dans le domaine visible et un faisceau d'éclairage monoculaire gauche dans le domaine infrarouge.

Avantageusement, le dispositif de multiplexage binoculaire comprend une pupille de sortie droite, une pupille de sortie gauche et des moyens optiques d'imagerie, ladite pupille de sortie droite étant apte à recevoir ledit faisceau d'éclairage monoculaire droit et ledit faisceau de mesure oculaire droit, respectivement ladite pupille de sortie gauche étant apte à recevoir ledit faisceau d'éclairage monoculaire gauche et ledit faisceau de mesure oculaire gauche, et lesdits moyens optiques d'imagerie étant aptes à former l'image d'une pupille de sortie dudit instrument ophtalmologique à voie unique de mesure sur ladite pupille de sortie droite et/ou sur ladite pupille de sortie gauche.

Avantageusement, le dispositif de multiplexage binoculaire comprend des moyens d'éclairage secondaire aptes à émettre un faisceau d'éclairage secondaire en direction de la cornée de chaque œil de manière à générer un faisceau de mesure oculaire droit, et respectivement gauche, pour délivrer une analyse de la cornée par kératométrie et/ou topographie cornéenne.

Avantageusement, le dispositif de multiplexage binoculaire comprend en outre un système optique disposé sur le trajet optique du faisceau image de stimulation, ledit système optique étant apte à compenser un défaut de sphère moyen des deux yeux.

Avantageusement, le dispositif de multiplexage binoculaire comprend en outre un premier système optique disposé sur le trajet optique du faisceau de stimulation oculaire droit et un second système optique disposé sur le trajet optique du faisceau de stimulation oculaire gauche, ledit premier, respectivement second, système optique étant apte à compenser un défaut de sphère et/ou de cylindre de l'œil droit, respectivement gauche, du sujet.

Selon des aspects particuliers et avantageux du dispositif de multiplexage binoculaire selon l'invention, le dispositif comprend :
- un système optique droit disposé entre les premiers moyens de combinaison optique et l'œil droit du sujet,
- un système optique gauche disposé entre les seconds moyens de combinaison optique et l'œil gauche du sujet, et
- des moyens d'alignement dudit système optique droit et dudit système optique gauche de manière à ajuster l'écartement pupillaire en fonction de la convergence prismatique du sujet.

Selon des aspects particuliers et avantageux du dispositif de multiplexage binoculaire selon l'invention, le dispositif comprend une unité de traitement électronique adaptée à recevoir un signal représentatif de l'état de commutation dudit commutateur optique et à recevoir séquentiellement une première mesure représentative dudit faisceau de mesure oculaire droit et une deuxième mesure représentative dudit faisceau de mesure oculaire gauche, ladite unité de traitement électronique étant apte à combiner ledit signal représentatif de l'état de commutation et lesdites première et deuxième mesures pour délivrer une mesure binoculaire multiplexée d'au moins un paramètre de vision.

L'invention propose également un procédé de détermination d'au moins un paramètre de vision binoculaire d'un sujet, basé sur l'utilisation d'un dispositif de multiplexage binoculaire combiné à un instrument ophtalmologique selon la revendication 1, ledit procédé comprenant les étapes suivantes :
a) envoi d'un faisceau image de stimulation pour une valeur de proximité P ;
b) séparation spatiale du faisceau image de stimulation en un faisceau de stimulation oculaire droit et un faisceau de stimulation oculaire gauche de manière à stimuler simultanément une accommodation de l'œil droit et de l'œil gauche du sujet ;
c) génération d'un faisceau d'éclairage monoculaire droit et/ou d'un faisceau d'éclairage monoculaire gauche, dans lequel l'étape c) de génération d'un faisceau d'éclairage monoculaire droit et d'un faisceau d'éclairage monoculaire gauche est réalisée en divisant un faisceau d'éclairage binoculaire de manière à éclairer simultanément l'œil droit et l'œil gauche du sujet ;
d) superposition optique dudit faisceau de stimulation oculaire droit et dudit faisceau d'éclairage monoculaire droit sur une voie optique droite et/ou respectivement superposition optique dudit faisceau de stimulation oculaire gauche et dudit faisceau d'éclairage monoculaire gauche sur une voie optique gauche ;
e) collection séquentielle via le commutateur optique du faisceau de mesure monoculaire droit ou respectivement du faisceau de mesure monoculaire gauche suivant un chemin optique séparé du faisceau d'éclairage monoculaire droit, respectivement gauche ;
f) analyse du faisceau de mesure monoculaire droit ou respectivement du faisceau de mesure monoculaire gauche, de manière à déterminer au moins un paramètre de vision monoculaire droit, ou respectivement gauche,
g) répétition des étapes c) à f) en commutant le faisceau de mesure monoculaire de manière à déterminer au moins un paramètre de vision binoculaire d'un sujet pour une même stimulation oculaire de l'œil droit et de l'œil gauche du sujet.

Selon des aspects particuliers et avantageux, le procédé de détermination d'au moins un paramètre de vision binoculaire objective d'un sujet selon l'invention, comprend les étapes du procédé ci-dessus pour une première valeur de proximité P1, puis les étapes du même procédé pour au moins une autre valeur de proximité Pn.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement un instrument ophtalmologique de mesure binoculaire multiplexée selon un premier mode de réalisation non revendiqué ;
- la figure 2 représente schématiquement un instrument ophtalmologique de mesure binoculaire multiplexée selon un mode de réalisation de l'invention ;
- la figure 3 représente schématiquement un instrument ophtalmologique de mesure binoculaire multiplexée selon une variante du premier mode de réalisation non revendiqué ;
- la figure 4 représente schématiquement un dispositif de multiplexage binoculaire selon un deuxième mode de réalisation non revendiqué ;
- la figure 5 représente schématiquement un dispositif de multiplexage binoculaire selon un troisième mode de réalisation non revendiqué ;
- la figure 6 représente schématiquement un dispositif de multiplexage binoculaire selon une variante du troisième mode de réalisation non revendiqué.

De manière générale, il est souhaitable de mesurer précisément les paramètres de compensation tels que sphère, cylindre, axe, aberrations d'ordres supérieurs (cf. norme ISO 24157:2008 qui spécifie les méthodes normalisées permettant de consigner les aberrations de l'œil humain), kératométrie, topographie cornéenne, diamètre de pupille ... généralement dans des conditions de vision binoculaire.

Nous allons maintenant détailler différents modes de réalisation du dispositif qui permettent une mesure binoculaire.

Le principe est de traiter différemment les voies optiques du stimulus visuel et de la mesure, afin de rendre binoculaire un unique stimulus et de sélectionner un œil à mesurer alternativement sans déplacement mécanique de la totalité de l'instrument de mesure.

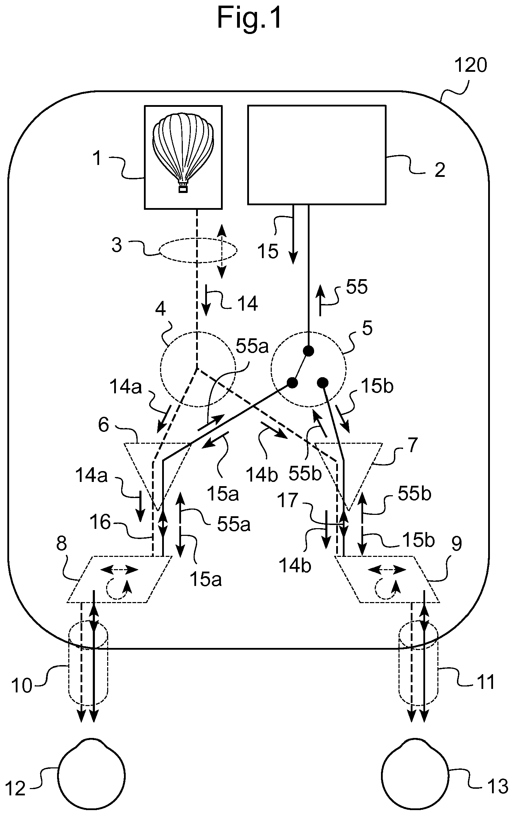

Sur la figure 1, on a représenté en vue de dessus un instrument ophtalmologique de mesure binoculaire multiplexée selon un premier mode de réalisation non revendiqué. L'instrument ophtalmologique 120 de la figure 1 intègre dans un même boîtier (représenté par un trait fin) un stimulus visuel 1, un moyen de mesure 2 et un système de multiplexage-démultiplexage binoculaire. Dans la suite du présent document, on utilise par simplification le terme système de multiplexage binoculaire. L'instrument ophtalmologique binoculaire comprend un oculaire droit 11 disposé face à l'œil droit 13 du sujet et un oculaire gauche 10 disposé face à l'œil gauche 12 du sujet. Sur la voie de sortie vers l'œil droit, l'instrument comporte un moyen de réglage 9 par déplacement latéral et par orientation. De même, sur la voie de sortie vers l'œil gauche, l'instrument comporte un moyen de réglage 8 par déplacement latéral et par orientation. Les moyens de réglage 8 et 9 permettent de régler le demi-écart pupillaire droit, respectivement gauche. Avantageusement, les moyens de réglage 8 et 9 permettent un mouvement à la fois latéral et angulaire de l'axe optique gauche 16 et respectivement de l'axe optique droite 17, afin de régler le système en fonction de l'écart inter-pupillaire et de la convergence prismatique en vision de près. En option, le dispositif peut comporter des moyens de réglage pour aligner les faisceaux en fonction de la hauteur de chaque œil.

Nous allons maintenant détailler le système de stimulation binoculaire de l'instrument ophtalmologique de mesure binoculaire de la figure 1. Le système de stimulation binoculaire comporte une mire de stimulus 1 unique et commune pour les deux yeux. La mire de stimulus 1 est éclairée par une source lumineuse de manière à générer un faisceau de stimulation 14 unique. Un système de conjugaison optique 3 contribue à former un faisceau image de stimulation 14 vers les yeux du sujet 12 et 13. Avantageusement, le système de conjugaison optique 3 a une puissance optique variable, de manière à modifier la proximité de la mire de stimulus 1 et à stimuler l'accommodation visuelle du sujet pour une valeur de proximité prédéterminée. Le système de stimulation binoculaire comporte aussi un séparateur de faisceau 4 disposé entre d'une part la mire de stimulus 1 et d'autre part les moyens de réglage 8 et 9 décrits plus haut. Le séparateur de faisceau 4 est apte à séparer spatialement le faisceau image de stimulation 14 en un faisceau de stimulation oculaire droit 14b et un faisceau de stimulation oculaire gauche 14a. Avantageusement, les deux faisceaux de stimulation oculaire droit 14b et gauche 14a ont une même intensité optique.

D'autre part, l'instrument ophtalmologique de la figure 1 comporte un moyen de mesure 2 ophtalmologique configuré pour l'analyse des caractéristiques optiques d'un seul œil à la fois. Le moyen de mesure 2 comporte une source lumineuse apte à émettre un faisceau optique d'éclairage 15 et un capteur apte à détecter un faisceau optique 55 de réflexion et/ou de réfraction. Le principe de la mesure ophtalmologique repose sur la mesure d'un faisceau 55 de réflexion et/ou de réfraction du faisceau optique d'éclairage 15 unique par l'œil droit 13 ou par l'œil gauche 12. Avantageusement, le faisceau optique d'éclairage 15 unique et le faisceau optique 55 de réflexion et/ou de réfraction sont colinéaires à l'entrée du moyen de mesure 2.

L'instrument de mesure ophtalmologique comporte en outre un commutateur optique 5 ayant au moins deux positions. Le commutateur optique 5 disposé entre d'une part le moyen de mesure 2 et d'autre part les moyens de réglage 8 et 9 décrits plus haut. Le commutateur optique 5 est apte à recevoir le faisceau d'éclairage unique 15 et à former un faisceau d'éclairage monoculaire droit 15b dirigé vers l'œil droit 13 du sujet, ou sélectivement à former un faisceau d'éclairage monoculaire gauche 15a dirigé vers l'œil gauche 12 du sujet (comme représenté sur la figure 1). Le commutateur optique 5 fonctionne dans les deux sens de propagation des faisceaux optiques. Lorsque le commutateur 5 est dans une position d'éclairage de l'œil gauche 12, le commutateur optique 5 est apte à recevoir un faisceau de mesure monoculaire gauche 55a issu de la réflexion et/ou de réfraction du faisceau d'éclairage par l'œil gauche 12 et à le transmettre vers le capteur du moyen de mesure 2 (cf. Figure 1). Lorsque le commutateur optique 5 est en position d'éclairage de l'œil droit 13, le commutateur 5 reçoit un faisceau de mesure monoculaire droit 55b issu de la réflexion et/ou de réfraction du faisceau d'éclairage par l'œil droit 13 et transmet le faisceau de mesure monoculaire droit 55b vers le capteur du moyen de mesure 2. Le commutateur optique 5 effectue ainsi le démultiplexage du faisceau d'éclairage et le multiplexage des faisceaux de mesure monoculaire droit 55b et gauche 55a en direction d'un même capteur. Le moyen de mesure 2 utilise une mesure monoculaire droite et une mesure monoculaire gauche pour en déduire une mesure binoculaire multiplexée.

L'instrument de mesure ophtalmologique comporte aussi un moyen optique de superposition 6 sur une voie optique de gauche 16 et respectivement un moyen optique de superposition 7 sur une voie optique de droite 17. Le moyen de superposition 7 est disposé entre le séparateur optique de faisceau 4, le commutateur optique 5 et l'oculaire droit 11. Le moyen de superposition 7 reçoit le faisceau de stimulation oculaire droit 14b et le faisceau d'éclairage monoculaire droit 15b et les superpose sur la voie optique de droite 17 en direction de l'œil droit 13. Dans l'autre sens de propagation, le moyen de superposition 7 reçoit le faisceau de mesure monoculaire droit 55b et le dirige vers le commutateur optique 5. De manière analogue, le moyen de superposition 6 est disposé entre le séparateur optique de faisceau 4, le commutateur optique 5 et l'oculaire gauche 10. Le moyen de superposition 6 reçoit le faisceau de stimulation oculaire gauche 14a et le faisceau d'éclairage monoculaire gauche 15a et les superpose sur la voie optique de gauche 16 en direction de l'œil gauche 12. Dans l'autre sens de propagation, le moyen de superposition 6 reçoit le faisceau de mesure monoculaire gauche 55a et le dirige vers le commutateur optique 5. On observe que le faisceau de stimulation oculaire 14, 14a, 14b ne passe pas par le commutateur optique 5. Les moyens de superposition optique 6 et 7 permettent d'assurer que les deux yeux sont en permanence stimulés par les faisceaux de stimulation oculaire droit 14b et gauche 14a, tandis que la mesure de réflexion et/ou réfraction est effectuée pour un seul œil à la fois, en fonction de la position du commutateur optique 5. De cette manière, la stimulation visuelle est toujours binoculaire tandis que les deux mesures monoculaires gauche et droite sont effectuées l'une après l'autre.

Le système optique 3, disposé entre la mire de stimulus 1 et le séparateur de faisceau 4, permet par exemple de compenser l'erreur moyenne de sphère des deux yeux 12, 13 afin d'améliorer la netteté et la fusion du stimulus. Avantageusement, le système optique 3 a pour effet de rendre la taille angulaire du stimulus constante et indépendante de la vergence.

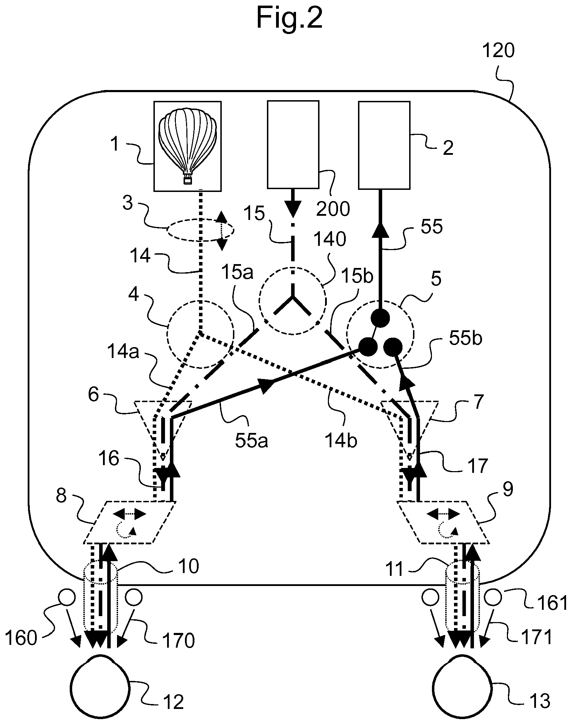

Sur la figure 2, on a représenté en vue de dessus un instrument ophtalmologique de mesure binoculaire multiplexée selon un mode de réalisation de l'invention. Les mêmes éléments sont représentés par les mêmes signes de référence que sur la figure 1. L'instrument ophtalmologique 120 de la figure 2 intègre dans un même boîtier (représenté par un trait fin) un stimulus visuel 1, un moyen de mesure 2, un moyen d'éclairage 200 et un système de multiplexage binoculaire. L'instrument ophtalmologique binoculaire comprend un oculaire droit 11 disposé face à l'œil droit 13 du sujet et un oculaire gauche 10 disposé face à l'œil gauche 12 du sujet. Sur la voie de sortie vers l'œil droit, l'instrument comporte un moyen de réglage 9 par déplacement latéral et par orientation. De même, sur la voie de sortie vers l'œil gauche, l'instrument comporte un moyen de réglage 8 par déplacement latéral et par orientation. Les moyens de réglage 8 et 9 permettent de régler le demi-écart pupillaire droit, respectivement gauche. Avantageusement, les moyens de réglage 8 et 9 permettent un mouvement à la fois latéral et angulaire de la voie optique gauche 16 et respectivement de la voie optique droite 17, afin de régler le système en fonction de l'écart inter-pupillaire et de la convergence prismatique en vision de près, ainsi que des hauteurs des deux yeux.

Nous allons maintenant détailler le système de stimulation binoculaire de l'instrument ophtalmologique de mesure binoculaire de la figure 2. Le système de stimulation binoculaire comporte une mire de stimulus 1 unique et commune pour les deux yeux. La mire de stimulus 1 est éclairée par une source lumineuse de manière à générer un faisceau de stimulation 14 unique. Un système de conjugaison optique 3 contribue à former un faisceau image de stimulation 14 vers les yeux du patient 12 et 13. Avantageusement, le système de conjugaison optique 3 a une puissance optique variable, de manière à modifier la proximité de la mire de stimulus 1 et à stimuler l'accommodation visuelle du sujet pour une valeur de proximité prédéterminée. Le système de stimulation binoculaire comporte aussi un séparateur de faisceau 4 disposé entre d'une part la mire de stimulus 1 et d'autre part les moyens de réglage 8 et 9 décrits plus haut. Le séparateur de faisceau 4 est apte à séparer spatialement le faisceau image de stimulation 14 en un faisceau de stimulation oculaire droit 14b et un faisceau de stimulation oculaire gauche 14a. Avantageusement, les deux faisceaux de stimulation oculaire droit 14b et gauche 14a ont une même intensité optique.

D'autre part, l'instrument ophtalmologique de la figure 2 comporte une source lumineuse d'éclairage 200 apte à émettre un faisceau optique d'éclairage binoculaire 15. Le système d'éclairage binoculaire comporte aussi un séparateur de faisceau 140 disposé entre d'une part la source lumineuse d'éclairage 200 et d'autre part les moyens de réglage 8 et 9 décrits plus haut. Le séparateur de faisceau 140 est apte à séparer spatialement le faisceau d'éclairage 15 en un faisceau de d'éclairage oculaire droit 15b et un faisceau d'éclairage oculaire gauche 15a. Avantageusement, les deux faisceaux d'éclairage oculaire droit 15b et gauche 15a ont une même intensité optique. Les deux faisceaux d'éclairage oculaire droit 15b et gauche 15a sont aptes à éclairer les deux yeux simultanément, alors que la mesure n'est effectuée que sur un œil à la fois.

D'autre part, l'instrument ophtalmologique de la figure 2 comporte un moyen de mesure 2 ophtalmologique configuré pour l'analyse des caractéristiques optiques d'un seul œil à la fois. Le moyen de mesure 2 un capteur apte à détecter un faisceau optique 55 de réflexion et/ou de réfraction. Le principe de la mesure ophtalmologique repose sur la mesure d'un faisceau 55 de réflexion et/ou de réfraction du faisceau optique d'éclairage 15 vu par les deux yeux 12 et 13.

L'instrument de mesure ophtalmologique comporte en outre un commutateur optique 5 ayant au moins deux positions. Lorsque le commutateur 5 est dans une position de mesure de l'œil gauche 12, le commutateur optique 5 est apte à recevoir un faisceau de mesure monoculaire 55a provenant de l'œil gauche 12 et à le transmettre vers le capteur du moyen de mesure 2 (cf. Figure 2). Lorsque le commutateur optique 5 est en position de mesure de l'œil droit 13, le commutateur 5 reçoit un faisceau de mesure 55b de l'œil droit 13 et transmet le faisceau de mesure monoculaire droit 55b vers le capteur du moyen de mesure 2. Le commutateur optique 5 effectue le multiplexage des faisceaux de mesure monoculaire droit 55b et gauche 55a en direction d'un même capteur. Le moyen de mesure 2 utilise une mesure monoculaire droite et une mesure monoculaire gauche pour en déduire une mesure binoculaire multiplexée.

L'instrument de mesure ophtalmologique comporte aussi un moyen optique de superposition 6 sur une voie optique de gauche 16 et respectivement un moyen optique de superposition 7 sur une voie optique de droite 17. Le moyen de superposition 7 est disposé entre le séparateur optique de faisceau de stimulation 4, le séparateur optique de faisceau d'éclairage 140, le commutateur optique 5 et l'oculaire droit 11. Le moyen de superposition 7 reçoit le faisceau de stimulation oculaire droit 14b et le faisceau d'éclairage oculaire droit 15b et les superpose sur la voie optique de droite 17 en direction de l'œil droit 13. Dans l'autre sens de propagation, le moyen de superposition 7 reçoit le faisceau de mesure monoculaire droit 55b et le dirige vers le commutateur optique 5. De manière analogue, le moyen de superposition 6 est disposé entre le séparateur optique de faisceau de stimulation 4, le séparateur optique de faisceau d'éclairage 140, le commutateur optique 5 et l'oculaire gauche 10. Le moyen de superposition 6 reçoit le faisceau de stimulation oculaire gauche 14a et le faisceau d'éclairage oculaire droit 15b et les superpose sur la voie optique de gauche 16 en direction de l'œil gauche 12. Dans l'autre sens de propagation, le moyen de superposition 6 reçoit le faisceau de mesure monoculaire gauche 55a et le dirige vers le commutateur optique 5. On observe que le faisceau de stimulation oculaire 14, 14a, 14b ainsi que le faisceau d'éclairage 15, 15a, 15b ne passent pas par le commutateur optique 5. Les moyens de superposition optique 6 et 7 permettent d'assurer que les deux yeux sont en permanence stimulés et éclairés par les faisceaux de stimulation oculaire droit 14b et gauche 14a et les faisceaux d'éclairage droit 15b et gauche 15a, tandis que la mesure de réflexion et/ou réfraction est effectuée pour un seul œil à la fois, en fonction de la position du commutateur optique 5. De cette manière, la stimulation visuelle et l'éclairage sont toujours binoculaires tandis que les deux mesures monoculaires gauche et droite sont effectuées l'une après l'autre.

Le système optique 3, disposé entre la mire de stimulus 1 et le séparateur de faisceau 4, permet par exemple de compenser l'erreur moyenne de sphère des deux yeux 12, 13 afin d'améliorer la netteté et la fusion du stimulus. Avantageusement, le système optique 3 a pour effet de rendre la taille angulaire du stimulus constante et indépendante de la vergence.

De plus, le système de la figure 2 peut être équipé sur les pupilles de sorties gauche 10 et droite 11 de sources d'éclairage respectivement 160 et 161 pour faire une analyse de la cornée, comme par exemple de la kératométrie ou topographie cornéenne. Ces sources d'éclairage 160 et 161 émettent un faisceau lumineux respectivement 170 et 171 en direction de la cornée de chaque œil 12 et 13. La mesure est faite par le moyen de mesure 2 à travers les faisceaux de mesure 55a et 55b en fonction de l'état du commutateur 5.

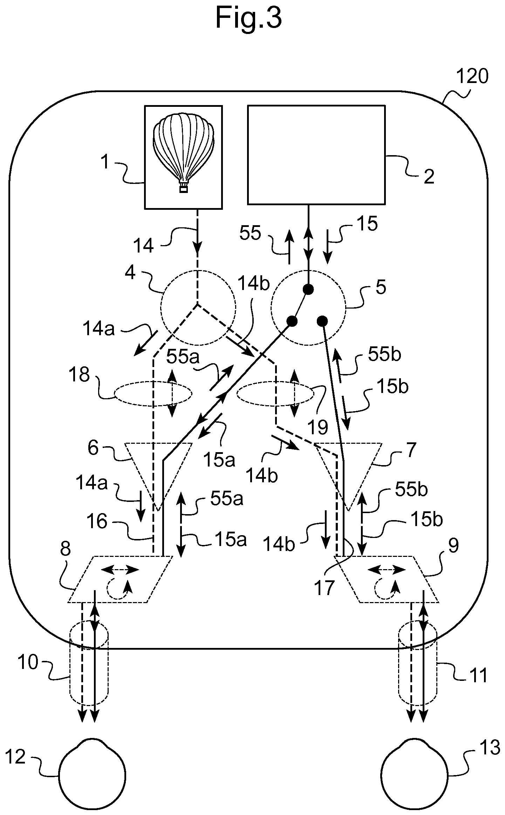

Sur la figure 3, on a représenté une deuxième variante de l'instrument de mesure ophtalmologique de la figure 1. Dans cette deuxième variante on a remplacé le système optique 3 placé devant la mire de stimulus 1 par deux systèmes optiques 18 et 19. Le système optique 18 est disposé entre le séparateur optique de faisceau 4 et le moyen optique de superposition 6 sur la voie optique de gauche. De manière symétrique, le système optique 19 est disposé entre le séparateur optique de faisceau 4 et le moyen optique de superposition 7 sur la voie optique de droite. Les systèmes optiques 18 et 19 permettent de compenser indépendamment l'erreur de sphère et/ou de cylindre de chaque œil. Avantageusement, les systèmes optiques 18 et 19 permettent aussi de corriger les décalages de distance optique créés par les moyens optiques 8 et 9 lors du réglage inter-pupillaire et de la convergence prismatique.

L'instrument de mesure ophtalmologique binoculaire multiplexé peut s'adapter à un large panel d'appareils de mesure ophtalmologique monoculaire existant. Le commutateur optique 5 permet un changement rapide et discret de la voie de mesure entre les deux yeux. Premièrement, l'écart pupillaire est ajustable. De plus, la stimulation est en permanence binoculaire. La mesure de réflexion et/ou de réfraction est effectuée pour chaque œil dans son axe de visée. La convergence du regard est ainsi prise en compte, par exemple lors d'une mesure en vision de près.

Le traitement séparé entre la voie du stimulus (visible) et la voie de mesure (infrarouge) évite l'utilisation d'optique spécifique dans une bande spectrale.

Un autre mode de réalisation de l'invention utilise le stimulus monoculaire et la mesure monoculaire d'un appareil extérieur. L'appareil monoculaire est combiné à un dispositif de multiplexage binoculaire pour transformer le stimulus monoculaire en stimulus binoculaire et pour sélectionner l'œil à mesurer.

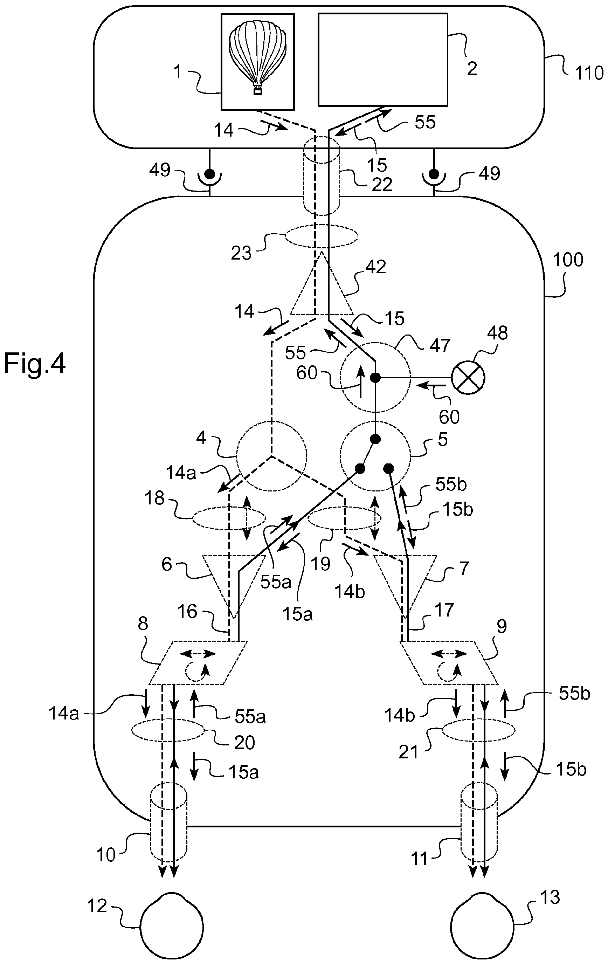

La figure 4 représente schématiquement un appareil monoculaire 110 couplé à un dispositif de multiplexage binoculaire 100 selon un deuxième mode de réalisation non revendiqué. Sur la figure 4, les mêmes éléments que ceux décrits en lien avec les figures 1 à 3 portent les mêmes signes de référence. L'appareil extérieur 110 comporte une mire de stimulus 1 et un moyen de mesure 2 et au moins une voie optique 22. Le moyen de mesure 2 comporte une source lumineuse apte à émettre un faisceau optique d'éclairage 15 et un capteur apte à détecter un faisceau optique 55 de réflexion et/ou de réfraction. La mire de stimulus 1 est éclairée par une source lumineuse de manière à générer un faisceau de stimulation 14 unique. La voie optique 22 est apte à faire passer le faisceau de stimulation 14, le faisceau d'éclairage 15 et à recevoir le faisceau de mesure de réflexion et/ou réfraction 55. Le dispositif de multiplexage binoculaire 100 comprend un oculaire droit 11, un oculaire gauche 10, un moyen de réglage 9 par déplacement latéral et par orientation sur la voie de sortie vers l'œil droit, un moyen de réglage 8 par déplacement latéral et par orientation sur la voie de sortie vers l'œil gauche. Le dispositif de multiplexage binoculaire 100 comporte aussi :
- un séparateur de faisceau 4 pour séparer spatialement un faisceau image de stimulation 14 unique en un faisceau de stimulation oculaire droit 14b et un faisceau de stimulation oculaire gauche 14a ;
- un commutateur optique 5 pour orienter la mesure sélectivement vers l'œil droit ou vers l'œil gauche ;
- un moyen optique de combinaison de faisceau 6 sur la voie optique de gauche 16 et respectivement un moyen optique de combinaison de faisceau 7 sur la voie optique de droite 17.

Le commutateur optique 5 et les moyens optiques de superposition 6 et 7 remplissent les mêmes fonctions et opèrent de manière analogue aux éléments correspondants décrits en lien avec la figure 1. Le dispositif de multiplexage binoculaire 100 comporte en outre :
- un séparateur optique de faisceau 42 disposé entre la voie optique 22 de l'appareil extérieur d'une part, le commutateur optique 5 et le premier séparateur de faisceau 4 d'autre part ;
- un système optique d'entrée 23, un système optique de sortie droite 21 et un système optique de sortie gauche 20,
- une mire d'alignement 48 et un autre séparateur de faisceau 47 disposés entre le séparateur de faisceau 42 et le commutateur optique 5,
- des attaches 49 de maintien entre l'instrument de mesure extérieur 110 et le dispositif de multiplexage binoculaire 100, lorsque l'alignement optique est réalisé ;
- un système d'appui frontal et une mentonnière pour maintenir la tête du sujet dans une posture déterminée, ou des points d'attache pour recevoir un calibre (oeil artificiel) et pour être compatible avec appareils existants.

Le séparateur de faisceau 42 permet de séparer le faisceau image de stimulation 14 unique issu de la mire de stimulus 1 et le faisceau d'éclairage 55 issu du système de mesure 2, ces deux faisceaux 14 et 22 provenant de la même voie optique 22. Le système optique d'entrée 23 est disposé entre la voie optique 22 et le séparateur de faisceau 42. Le système optique de sortie droite 21 est disposé entre les moyens optiques de réglage 9 et la voie optique de sortie droite 11. Le système optique de sortie gauche 20 est disposé entre les moyens optiques de réglage 8 et la voie optique de sortie gauche 10. La combinaison des systèmes optiques 23, 20 et 21 permet de former une imager des yeux 12, 13 au niveau de la voie optique d'entrée 22 à travers les voies optiques de sortie 10, 11. Avantageusement, la mire d'alignement peut être un calibre de type œil artificiel, dont on connaît les valeurs de réfraction. La mire d'alignement 48 émet un faisceau optique d'alignement 60. La mire d'alignement 48 est imagée sur la voie optique de mesure de l'appareil externe grâce aux séparateurs de faisceaux 47 et 42. La mire d'alignement 48 sert à régler la position de la pupille de sortie de l'appareil externe 110 en face de la pupille d'entrée du dispositif de multiplexage binoculaire 100. La mire d'alignement 48 sert aussi de référence de centrage lors du réglage des écarts pupillaires. La mire d'alignement 48 émet un faisceau optique d'alignement 60 uniquement pendant le réglage optique entre l'appareil externe de mesure et le dispositif de multiplexage. Pendant les mesures de réflexion et/ou de réfraction oculaire, la mire d'alignement 48 est éteinte.

Un dispositif de multiplexage binoculaire tel que représenté sur la figure 4 présente de nombreux avantages. Premièrement, le dispositif de multiplexage binoculaire 100 s'adapte à un large panel d'appareils monoculaires existants sans requérir de modification de l'appareil de mesure monoculaire 110. Comme détaillé plus haut, le dispositif de multiplexage binoculaire 100 permet un changement rapide et discret de la voie de mesure entre les deux yeux. De plus, chaque œil est mesuré suivant son axe de visée, ce qui permet de prendre en compte la convergence du regard. Enfin, l'écart pupillaire est ajustable. Avantageusement, le traitement séparé entre la voie du stimulus (visible) et la voie de mesure (infrarouge) évite l'utilisation de composants optiques spécifiques dans une bande spectrale limitée.

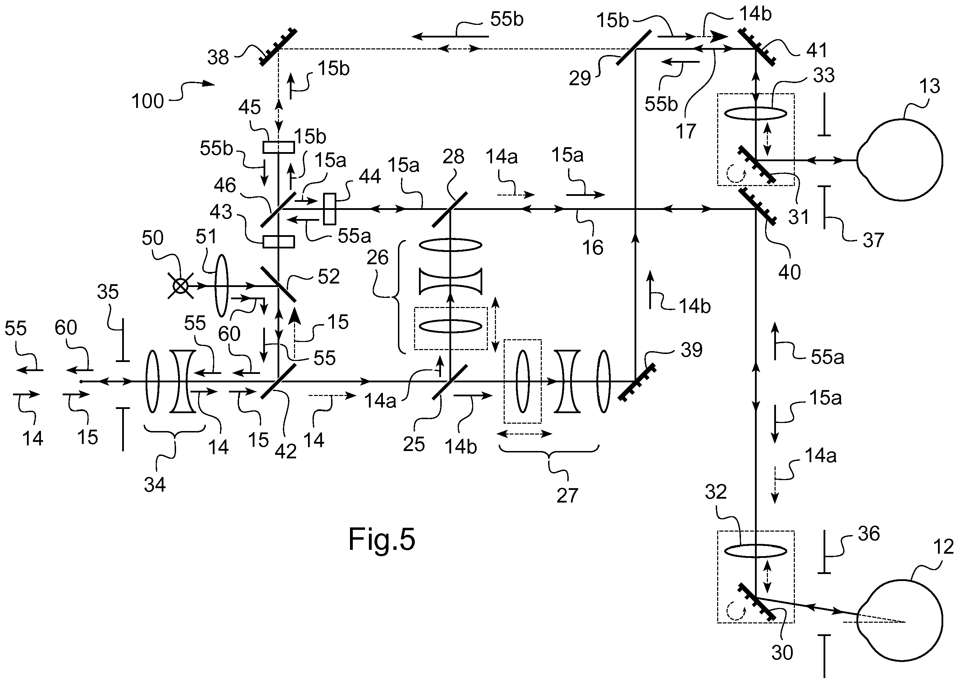

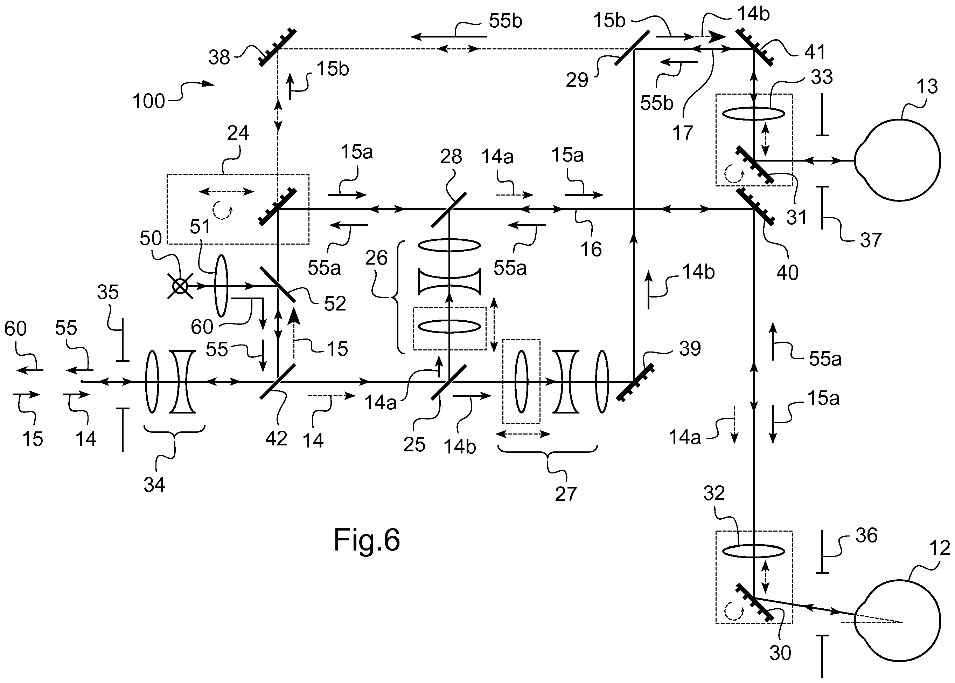

Les figures 5 et 6 représentent schématiquement un dispositif de multiplexage binoculaire 100 selon deux variantes d'un troisième mode de réalisation non revendiqué. Le dispositif de multiplexage binoculaire 100 de la figure 5 ou de la figure 6 est destiné à être disposé face à la voie optique d'un appareil de mesure monoculaire, comprenant une mire de stimulus et un moyen de mesure. Sur les figures 5 et 6, la mire de stimulus et les moyens de mesure ne sont pas représentés. Le dispositif de multiplexage binoculaire 100 comporte une pupille d'entrée 35 qui est destinée à être connectée à un appareil extérieur de mesure monoculaire. En entrée du dispositif de multiplexage binoculaire 100 est placé un premier système optique 34. Le système optique 34 est apte à recevoir un faisceau image de stimulation 14 et/ou un faisceau d'éclairage monoculaire 15 en provenance de l'appareil externe de mesure monoculaire. Dans l'autre sens, le système optique 34 est apte à recevoir un faisceau de mesure 55 en provenance de l'œil soit droit soit gauche du sujet et à transmettre ce faisceau de mesure vers l'appareil externe de mesure monoculaire. Une première lame séparatrice 42 est disposée sur le trajet optique des faisceaux de stimulation 14 et d'éclairage 15 en aval du système optique 34. La première lame séparatrice 42 sépare spatialement les voies optiques du stimulus et de la mesure. Dans l'exemple de réalisation représenté sur la figure 5, le faisceau de stimulation 14 traverse la lame séparatrice 42 alors que le faisceau d'éclairage 15 est réfléchi par la lame séparatrice 42. Avantageusement, la lame séparatrice 42 est une lame dichroïque, ayant un traitement de surface apte à séparer d'une part le faisceau de stimulation 14 dans le visible (entre environ 400 et 700nm) et d'autre part le faisceau d'éclairage 15 et le faisceau de mesure 55 dans l'infrarouge (l'infrarouge proche étant entre environ 750nm et 1000 nm). On peut par exemple utiliser une lame chaude qui laisse passer le visible et qui réfléchit l'infrarouge. Alternativement, on peut utiliser une lame froide qui laisse passer l'infrarouge et qui réfléchit le visible. Une deuxième lame séparatrice 25 (ou un cube séparateur) est disposée sur le trajet optique du faisceau image de stimulation 14 en aval de la première lame séparatrice 42. La deuxième lame séparatrice 25 divise spatialement le faisceau image de stimulation 14 en un faisceau de stimulation oculaire droit 14b dirigé vers l'œil droit 13 et un faisceau de stimulation oculaire gauche 14a dirigé vers l'œil gauche 12 du sujet. Sur la voie de stimulation oculaire droite, respectivement gauche, un groupe de lentilles 27, respectivement 26, agencé selon un système optique de Badal, permet de compenser la vision du stimulus en fonction du défaut sphérique de l'œil droit, respectivement l'œil gauche. Avantageusement, la taille angulaire du stimulus est indépendante de sa position. On peut donc augmenter ou diminuer la distance (vergence et convergence) de l'œil sur le stimulus sans en changer la taille angulaire.

Sur la figure 5, un premier élément polarisant 43 et une troisième lame séparatrice 46 sont disposés sur le trajet optique du faisceau d'éclairage 15 en aval de la première lame séparatrice 42. L'élément polarisant 43 est un élément polarisant d'axe de polarisation variable apte à recevoir le faisceau d'éclairage 15 et à modifier l'axe de polarisation du faisceau transmis. Avantageusement, on utilise un élément polarisant 43 à cristaux liquides piloté électriquement, dont l'axe de polarisation est orientable par application d'une tension électrique. Alternativement, l'élément polarisant 43 peut être un polariseur monté à rotation. Dans le cas où la source lumineuse est polarisée, l'élément polarisant 43 peut être remplacé par un moyen apte à faire tourner l'axe de polarisation de la source. La troisième lame séparatrice 46 (ou un cube séparateur) est disposée sur le trajet optique du faisceau d'éclairage 15 en aval de l'élément polarisant 43. La troisième lame séparatrice 46 est apte à diviser le faisceau d'éclairage 15 et à former un faisceau d'éclairage monoculaire droit 15b destiné à éclairer l'œil droit 13 du sujet et/ou à former un faisceau d'éclairage monoculaire gauche 15a destiné à éclairer l'œil gauche 12 du sujet. Un deuxième élément polarisant 44 est disposé sur le trajet optique du faisceau d'éclairage monoculaire gauche 15a en aval du premier élément polarisant 43 et de la troisième lame séparatrice 46. Un troisième élément polarisant 45 est disposé sur le trajet optique du faisceau d'éclairage monoculaire droit 15b en aval du premier élément polarisant 43 et de la troisième lame séparatrice 46. Les axes de polarisation des deuxième et troisième éléments polarisants 44 et 45 sont croisés l'un par rapport à l'autre. Les deuxième et troisième éléments polarisants 44 et 45 sont par exemple des polariseurs linéaires. De manière alternative, un seul élément de séparation peut combiner les fonctions de séparation de la lame séparatrice 46 et de polarisation des éléments polarisants 44 et 45. Dans une première position de mesure, l'axe de polarisation du premier élément polarisant 43 est aligné sur l'axe de polarisation du deuxième élément polarisant 44 et croisé avec l'axe de polarisation du troisième élément polarisant 45. Dans une deuxième position de mesure, l'axe de polarisation du premier élément polarisant 43 est aligné sur l'axe de polarisation du troisième élément polarisant 45 et croisé avec l'axe de polarisation du deuxième élément polarisant 44. De cette manière, un seul des deux faisceaux d'éclairage monoculaire gauche 15a, ou respectivement droit 15b, est actif tandis que l'autre faisceau d'éclairage monoculaire est éteint. Autrement dit, en modifiant l'orientation de l'axe de polarisation de l'élément polarisant 43 pour l'aligner sur l'axe de l'un ou de l'autre élément polarisant 44 ou 45, on commute le faisceau d'éclairage d'un œil vers l'autre œil. Les éléments 43, 44, 45 et 46 forment ainsi un commutateur optique apte à sélectionner alternativement un des deux yeux pour une mesure monoculaire. On note que les faisceaux de stimulation 14, 14a, 14b ne passent pas par le dispositif de commutation constitué par les éléments 43, 44, 45 et 46. De ce fait, les faisceaux de stimulation oculaire gauche 14a et droit 14b sont en permanence actifs simultanément de manière à stimuler la vision binoculaire du sujet.

Sur la figure 6, en variante du dispositif de la figure 5, les éléments polarisant 43, 44, 45 et la troisième lame séparatrice 46 sont remplacés par un miroir 24 orientable de manière à sélectionner une des deux voies optiques de mesure.

Sur les figures 5 et 6, on dispose ainsi d'un faisceau de stimulation oculaire droit 14b et d'un faisceau de stimulation oculaire gauche 14a et d'un faisceau d'éclairage monoculaire droit 15b ou d'un faisceau d'éclairage monoculaire gauche 15a. Une quatrième lame séparatrice 28 est disposée sur le trajet optique du faisceau d'éclairage monoculaire gauche 15a en aval de la deuxième lame séparatrice 25 et sur le trajet optique du faisceau de stimulation oculaire gauche 14a en aval du deuxième élément polarisant 44. La quatrième lame séparatrice 28 permet de combiner le faisceau d'éclairage monoculaire gauche 15a et le faisceau de stimulation oculaire gauche 14a sur une même voie optique gauche 16. Dans un exemple de réalisation, la quatrième lame séparatrice 28 est une lame dichroïque ayant un traitement de surface adapté pour permettre de recombiner le faisceau de stimulation oculaire gauche 14a dans le domaine visible et le faisceau d'éclairage monoculaire gauche 15a dans le domaine infrarouge (et dans le sens inverse, pour transmettre le faisceau de mesure monoculaire gauche 55a sur le même trajet optique que le faisceau d'éclairage monoculaire gauche 15a). La lame séparatrice 28 peut être une lame froide ou une lame chaude comme explicité plus haut. De même, une cinquième lame séparatrice 29 est disposée sur le trajet optique du faisceau d'éclairage monoculaire droit 15b en aval de la deuxième lame séparatrice 25 et sur le trajet optique du faisceau de stimulation oculaire droit 14b en aval du troisième élément polarisant 45. La cinquième lame séparatrice 29 permet de combiner le faisceau d'éclairage monoculaire droit 15b et le faisceau de stimulation oculaire droit 14b sur une même voie optique droite 17. Dans un exemple de réalisation, la cinquième lame séparatrice 29 est aussi une lame froide ayant un traitement de surface adapté pour permettre de recombiner le faisceau de stimulation oculaire droit 14b dans le domaine visible et le faisceau d'éclairage monoculaire droit 15b dans le domaine infrarouge (et dans le sens inverse, pour transmettre le faisceau de mesure monoculaire droit 55b sur le même trajet optique que le faisceau d'éclairage monoculaire droit 15b). Avantageusement, le faisceau infrarouge est transmis par la lame séparatrice froide 28, respectivement 29, alors que le faisceau visible est réfléchi par la lame froide. La lame froide 28, respectivement 29, réunit ainsi l'axe optique du faisceau de stimulation oculaire et l'axe optique du faisceau de mesure de chaque œil. Cette combinaison permet d'effectuer la mesure dans l'axe de visée de chaque oeil, notamment en vision de près, le sujet étant dans une position de vision binoculaire puisque les deux faisceaux de stimulation oculaire droit 14b et gauche 14a sont simultanément vus par le sujet comme indiqué plus haut.

Des miroirs 40, 41 servent à replier le trajet optique des voies optiques recombinées 16, respectivement 17, de chaque œil. Un système optique composé d'une lentille 32 et d'un miroir 30 permet d'aligner la voie optique gauche 16 sur l'axe de visée de l'œil gauche 12. De manière analogue, un système optique composé d'une lentille 33 et d'un miroir 31 permet d'aligner la voie optique gauche 16 sur l'axe de visée de l'œil gauche 12. Les miroirs 30, 31 peuvent aussi servir à ajuster le demi-écart pupillaire. Les chemins optiques des deux voies 16 et 17 sont calibrés pour être de la même longueur optique. Les miroirs de sortie 30 et 31 peuvent être translatés et orientés pour que l'axe optique de mesure soit centré sur la pupille de chaque œil en tenant compte de l'écart pupillaire et de la convergence des yeux.

Afin d'imager chaque œil à la même position optique définie par l'appareil monoculaire, on utilise le système optique à lentilles 34 placé devant la pupille d'entrée 35 et une lentille auxiliaire 32, 33 placée devant une pupille de sortie 36, respectivement 37. Les lentilles auxiliaires 32, 33 se déplacent conjointement avec le miroir de sortie 30, 31 associé de manière à compenser les différences de chemin optique entre la voie optique droite 17 et la voie optique gauche 16.

Lorsque les yeux 12, 13 du sujet sont alignés face aux pupilles de sortie de l'appareil de multiplexage binoculaire, chaque œil 12, 13 reçoit en permanence un faisceau de stimulation oculaire respectivement 14a, 14b. En fonction de la position de l'élément polarisant 43, l'œil droit 13 reçoit un faisceau d'éclairage monoculaire droit 15b ou l'œil gauche 12 reçoit un faisceau d'éclairage monoculaire gauche 15a. Le faisceau d'éclairage monoculaire droit 15b est réfléchi et/ou réfracté par l'œil droit 13 et forme un faisceau de mesure oculaire droit 55b. Alternativement, le faisceau d'éclairage monoculaire gauche 15a est réfléchi et/ou réfracté par l'œil gauche 12 et forme un faisceau de mesure oculaire gauche 55a. Le faisceau de mesure oculaire droit 55b suit le même trajet optique que le faisceau d'éclairage monoculaire droit 15b sur la voie optique droite 17 depuis l'œil droit 13 jusqu'à la troisième lame séparatrice 46. De même, le faisceau de mesure oculaire gauche 55a suit le même trajet optique que le faisceau d'éclairage monoculaire gauche 15a sur la voie optique gauche 16 depuis l'œil gauche 12 jusqu'à la troisième lame séparatrice 46. En fonction de la position de l'élément polarisant 43, la voie optique de droite 17 ou la voie optique de gauche 16 est activée pour la mesure. Le système composé des éléments polarisants 43, 44 et 45 fonctionne de manière réciproque pour les faisceaux de mesure oculaire droite 55b et gauche 55a.

Rappelons que dans la première position de mesure, l'axe de polarisation du premier élément polarisant 43 est orienté parallèlement à l'axe de polarisation du deuxième l'élément polarisant 44 et perpendiculairement à l'axe de polarisation du troisième élément polarisant 45. Dans la deuxième position de mesure, l'axe de polarisation du premier élément polarisant 43 est orienté parallèlement à l'axe de polarisation du troisième élément polarisant 45 et perpendiculairement à l'axe de polarisation du deuxième l'élément polarisant 44. De cette manière, un seul des deux faisceaux mesure oculaire droite 55b, ou respectivement gauche 55a, est transmis tandis que l'autre faisceau d'éclairage monoculaire et que l'autre faisceau de mesure sont obturés. Autrement dit, en modifiant l'orientation de l'axe de polarisation de l'élément polarisant 43 pour l'aligner sur l'axe de l'un ou de l'autre élément polarisant 45 ou 44, on commute le faisceau de mesure oculaire droite 55b ou gauche 55a en direction de l'appareil de mesure monoculaire externe. Les éléments 43, 44, 45 et 46 commutent simultanément le faisceau d'éclairage et le faisceau de mesure. En sortie de l'élément polarisant 43, le faisceau de mesure 55 (qui correspond soit au faisceau de mesure oculaire droit 55b soit au faisceau de mesure oculaire gauche 55a) suit le chemin optique inverse du faisceau d'éclairage monoculaire 15 et est réfléchi par la première lame séparatrice 42 en direction de l'appareil externe. Le dispositif 100 permet ainsi de démultiplexer les voies de mesure droite et gauche.

Le dispositif de multiplexage binoculaire 100 de la figure 5 comporte également une mire d'alignement 50 infrarouge et une lentille 51 apte à générer un faisceau optique d'alignement 60 pour former une image de la mire d'alignement optique 50. Avantageusement, la mire d'alignement optique 50 peut comprendre un calibre de type œil artificiel, dont on connaît les valeurs de réfraction. Une sixième lame séparatrice 52 est disposée entre la première lame séparatrice 42 et le premier élément polarisant 43. La sixième lame séparatrice 52 recombine le faisceau optique d'alignement 60 sur la voie du faisceau d'éclairage unique 15 et les dirige vers la pupille d'entrée 35 du système de multiplexage binoculaire. Cette mire d'alignement 50 sert à régler la position de la pupille de sortie de l'appareil monoculaire externe en face de la pupille d'entrée 35 du démultiplexeur. L'image de la mire d'alignement 50 est conjuguée optiquement avec les yeux du sujet 12, 13 de façon à régler la mise au point de l'appareil monoculaire. L'image de la mire d'alignement 50 sert aussi de référence de centrage lors du réglage des écarts pupillaires. Avantageusement, les lentilles 32, 33, 34, 51 sur la voie de mesure (Infrarouge) sont traitées antireflet dans l'infrarouge pour éviter les reflets parasites.

D'autres solutions techniques peuvent être mises en œuvre pour démultiplexer la voie de mesure. Sur la figure 6 les mêmes éléments sont désignés par les mêmes signes de référence que sur la figure 5. En variante du dispositif de la figure 5, les éléments polarisant 43, 44, 45 et la troisième lame séparatrice 46 sont remplacés sur la figure 6 par un miroir 24 orientable de manière à sélectionner une des deux voies optiques de mesure. Selon d'autres variantes du même mode de réalisation, un autre élément opto-mécanique, optoélectronique, acoustique ou thermique peut également permettre de sélectionner une des deux voies dans le cadre de la présente divulgation.

### Mode opératoire et fonctionnement du système :

1. Le praticien positionne la pupille de sortie d'un appareil de mesure ophtalmologique monoculaire en face de la pupille d'entrée 35 du dispositif de multiplexage-démultiplexage binoculaire. Pour cela, il active la mire d'alignement 50, puis il déplace verticalement et horizontalement l'appareil monoculaire jusqu'à voir et centrer la mire d'alignement 50 du démultiplexeur sur son écran de contrôle. Le praticien règle ensuite la mise au point sur la mire qui représente la position virtuelle de l'œil. Les réglages finis, le praticien fixe le démultiplexeur à l'appareil monoculaire par les attaches 49 (ventouses, scratch, aimants...)
2. Le sujet se positionne devant les pupilles de sortie 36, 37 du dispositif de multiplexage-démultiplexage binoculaire. Le praticien règle alors les écarts pupillaires. Pour cela, la mire de stimulus 1 de l'appareil monoculaire est placée à l'infini afin d'éviter la convergence des yeux. Ensuite, le praticien sélectionne un œil à mesurer grâce au moyen de commutation optique 43 ou 24. Puis le praticien déplace l'ensemble lentille-miroir 30, 32 pour que la pupille de l'œil 12 soit centrée avec la mire d'alignement 50. La même opération est réitérée en déplaçant l'ensemble lentille-miroir 31, 33 pour l'autre œil 13.
3. Le praticien initialise les valeurs du paramètre ophtamologique recherché en saisissant une prescription ou une mesure précédente ou encore en faisant une première mesure en vision de loin. Un sujet amétrope ne voit pas le stimulus net lors de cette première mesure. Le praticien sélectionne un œil à mesurer grâce au moyen de commutation optique 43 ou 24, effectue la première mesure de l'œil sélectionné en vision de loin puis change d'œil et effectue la première mesure de l'autre œil en vision de loin. On obtient ainsi une première mesure binoculaire en vision de loin.
4. A l'aide de cette valeur initiale provenant d'une première mesure ou d'une mesure antérieure, le praticien corrige soit la sphère moyenne des deux yeux soit la sphère et/ou le cylindre de chaque œil grâce aux systèmes optiques de Badal et/ou cylindres croisés 26, 27.
5. Il est préférable pour le praticien de refaire une nouvelle mesure en vision de loin avec un stimulus net pour le sujet.
6. Pour la vision intermédiaire ou de près, l'image de la mire de stimulus est positionné à une distance optique connue permettant, en fonction de l'écart pupillaire du sujet, au système de se positionner dans l'axe de convergence prismatique de chaque œil en déplaçant et orientant les miroirs de sortie 30, 31. Le mode opératoire de la mesure binoculaire en vision intermédiaire ou en vision de près reste le même que pour la vision de loin.

Le dispositif et le procédé de multiplexage binoculaire de l'invention permettent de diffuser vers les deux yeux en même temps un même stimulus visuel, ce qui permet d'effectuer une mesure de réflexion et/ou réfraction oculaire tenant compte de la convergence du regard. Le dispositif ne mesure qu'un seul œil à la fois, mais est capable de basculer instantanément la voie optique de mesure d'un œil à l'autre œil. Avantageusement, le dispositif de l'invention comprend des moyens de réglage préalables du système en fonction de l'écart pupillaire du sujet. Dans le cas d'un stimulus et d'un moyen de mesure externes, le dispositif de multiplexage permet de séparer les deux voies pour les traiter indépendamment.

L'invention permet de mesurer la réfraction d'un œil avec une stimulation binoculaire à partir d'un unique moyen de mesure et d'un unique stimulus. Le dispositif de l'invention permet la mesure dans l'axe de visée de l'œil en tenant compte de la convergence prismatique en vision de près. Le dispositif de l'invention peut sélectionner l'œil à mesurer tout en maintenant un stimulus binoculaire et sans déplacement mécanique lors du changement d'œil.

L'invention est particulièrement adaptée pour toute personne qui pratique des mesures ophtalmologiques par réfraction et qui désire proposer ou effectuer des mesures binoculaires.

Le dispositif de l'invention peut être utilisé par un optométriste ou un ophtalmologiste, ou encore par un opticien pour déterminer les paramètres de personnalisation des verres de lunettes.

## Revendications

1. Dispositif de multiplexage binoculaire combiné à un instrument ophtalmologique (120) à plusieurs voies de mesure objective d'au moins un paramètre de vision d'un sujet, ledit instrument ophtalmologique comportant un moyen d'éclairage (200) apte à émettre un faisceau d'éclairage binoculaire (15), des moyens pour collecter un faisceau de mesure (55) par réflexion et/ou réfraction dudit faisceau d'éclairage sur un œil du sujet, et un capteur (2) associé à une voie unique de mesure, le moyen d'éclairage (200) étant séparé du capteur (2), ledit dispositif de multiplexage binoculaire comprenant :
- des premiers moyens de séparation optique (4) aptes à recevoir un faisceau image de stimulation (14) issu d'une mire de stimulus (1) et destiné à stimuler une accommodation du sujet, lesdits premiers moyens de séparation optique (4) étant aptes à séparer spatialement ledit faisceau image de stimulation (14) en un faisceau de stimulation oculaire droit (14b) et un faisceau de stimulation oculaire gauche (14a) de manière à stimuler simultanément une accommodation de l'œil droit (13) et de l'œil gauche (12) du sujet ;
- des seconds moyens de séparation optique (140) aptes à recevoir ledit faisceau d'éclairage binoculaire (15) et séparer ledit faisceau d'éclairage binoculaire (15) en un faisceau d'éclairage monoculaire droit (15b) et en un faisceau d'éclairage monoculaire gauche (15a), ledit faisceau d'éclairage monoculaire droit (15b), et respectivement ledit faisceau d'éclairage monoculaire gauche (15a), étant destinés à éclairer simultanément l'œil droit (13), respectivement l'œil gauche (12) du sujet pour former, après réflexion et/ou réfraction par l'œil concerné (12, 13), un faisceau de mesure oculaire droit (55b), respectivement gauche (55a),
- un commutateur optique (5) disposé en dehors du chemin optique du faisceau de stimulation oculaire droit (14b), du faisceau de stimulation oculaire gauche (14a), du faisceau d'éclairage monoculaire droit (15b) et du faisceau d'éclairage monoculaire gauche (15a), le commutateur optique (5) étant apte à recevoir ledit faisceau de mesure oculaire droit (55b) et respectivement ledit faisceau de mesure oculaire gauche (55a) et à acheminer séquentiellement ledit faisceau de mesure oculaire droit (55b) ou respectivement ledit faisceau de mesure oculaire gauche (55a) vers ledit capteur (2) associé à ladite voie unique de mesure, lorsque le faisceau d'éclairage monoculaire droit (15b) et respectivement le faisceau d'éclairage monoculaire gauche (15a) éclairent simultanément l'œil droit (13) et l'œil gauche (12) du sujet,
- des premiers moyens de combinaison optique (7) aptes à superposer le faisceau de stimulation oculaire droit (14b) et le faisceau d'éclairage monoculaire droit (15b) et des premiers moyens optiques pour diriger le faisceau de stimulation oculaire droit (14b) et le faisceau d'éclairage monoculaire droit (15b) vers l'œil droit (13), et
- des seconds moyens de combinaison optique (6) aptes à superposer le faisceau de stimulation oculaire gauche (14a) et le faisceau d'éclairage monoculaire gauche (15a) et des seconds moyens optiques pour diriger le faisceau de stimulation oculaire gauche (14a) et le faisceau d'éclairage monoculaire gauche (15a) vers l'œil gauche (12).

2. Dispositif de multiplexage binoculaire combiné à un instrument ophtalmologique selon la revendication 1, dans lequel lesdits premiers moyens de séparation optique (4) comprennent une lame séparatrice ou un cube séparateur de faisceau.

3. Dispositif de multiplexage binoculaire combiné à un instrument ophtalmologique selon la revendication 1 ou la revendication 2, dans lequel ledit commutateur optique (5) comprend une lame dichroïque, un miroir orientable, un miroir escamotable.

4. Dispositif de multiplexage binoculaire combiné à un instrument ophtalmologique selon l'une des revendications 1 à 3, dans lequel lesdits seconds moyens de séparation optique comprennent une lame séparatrice ou un cube séparateur.

5. Dispositif de multiplexage binoculaire combiné à un instrument ophtalmologique selon l'une des revendications 1 à 4, dans lequel lesdits premiers moyens de combinaison optique (7) comprennent une lame dichroïque apte à superposer un faisceau de stimulation oculaire droit (14b) dans le domaine visible et un faisceau d'éclairage monoculaire droit (15b) dans le domaine infrarouge, et/ou respectivement lesdits seconds moyens (6) de combinaison optique comprennent une lame dichroïque apte à superposer un faisceau de stimulation oculaire gauche (14a) dans le domaine visible et un faisceau d'éclairage monoculaire gauche (15a) dans le domaine infrarouge.

6. Dispositif de multiplexage binoculaire combiné à un instrument ophtalmologique selon l'une des revendications 1 à 5 comprenant une pupille de sortie droite (37), une pupille de sortie gauche (36) et des moyens optiques d'imagerie (20, 21, 23, 32, 33, 34) ladite pupille de sortie droite (37) étant apte à recevoir ledit faisceau d'éclairage monoculaire droit (15b) et ledit faisceau de mesure oculaire droit (55b), respectivement ladite pupille de sortie gauche (36) étant apte à recevoir ledit faisceau d'éclairage monoculaire gauche (15a) et ledit faisceau de mesure oculaire gauche (55a), et lesdits moyens optiques d'imagerie (18, 19, 20, 21, 23, 32, 33, 34) étant aptes à former l'image d'une pupille de sortie (22, 35) dudit instrument ophtalmologique à voie unique de mesure sur ladite pupille de sortie droite (37) et/ou sur ladite pupille de sortie gauche (36).

7. Dispositif de multiplexage binoculaire combiné à un instrument ophtalmologique selon la revendication 6 comprenant des moyens d'éclairage secondaire (160, 161) aptes à émettre un faisceau d'éclairage secondaire (170, 171) en direction de la cornée de chaque œil (12, 13) de manière à générer un faisceau de mesure oculaire droit (55b), et respectivement gauche (55a), pour délivrer une analyse de la cornée par kératométrie et/ou topographie cornéenne.

8. Dispositif de multiplexage binoculaire combiné à un instrument ophtalmologique selon l'une des revendications 1 à 7 comprenant en outre un système optique (3) disposé sur le trajet optique du faisceau image de stimulation (14), ledit système optique (3) étant apte à compenser un défaut de sphère moyen des deux yeux (12, 13).

9. Dispositif de multiplexage binoculaire combiné à un instrument ophtalmologique selon l'une des revendications 1 à 8 comprenant en outre un premier système optique (18, 26) disposé sur le trajet optique du faisceau de stimulation oculaire droit (14b) et un second système optique (19, 27) disposé sur le trajet optique du faisceau de stimulation oculaire gauche (14a), ledit premier, respectivement second, système optique (18, 26, 19, 27) étant apte à compenser un défaut de sphère et/ou de cylindre de l'œil droit (13), respectivement gauche (12), du sujet.

10. Dispositif de multiplexage binoculaire combiné à un instrument ophtalmologique selon l'une des revendications 1 à 9 comprenant :
- un système optique droit (9, 11, 21) disposé entre les premiers moyens de combinaison optique (7) et l'œil droit (13) du sujet,
- un système optique gauche (8, 10, 20) disposé entre les seconds moyens de combinaison optique (6) et l'œil gauche (12) du sujet, et
- des moyens d'alignement (30, 31, 32, 33) dudit système optique droit (9, 11, 21, 33) et dudit système optique gauche (8, 10, 20, 32) de manière à ajuster l'écartement pupillaire en fonction de la convergence prismatique du sujet.

11. Dispositif de multiplexage binoculaire combiné à un instrument ophtalmologique selon l'une des revendications 1 à 10 comprenant une unité de traitement électronique adaptée à recevoir un signal représentatif de l'état de commutation dudit commutateur optique (5) et à recevoir séquentiellement une première mesure représentative dudit faisceau de mesure oculaire droit (55b) et une deuxième mesure représentative dudit faisceau de mesure oculaire gauche (55a), ladite unité de traitement électronique étant apte à combiner ledit signal représentatif de l'état de commutation et lesdites première et deuxième mesures pour délivrer une mesure binoculaire multiplexée d'au moins un paramètre de vision.

12. Procédé de détermination d'au moins un paramètre de vision binoculaire d'un sujet, basé sur l'utilisation d'un dispositif de multiplexage binoculaire combiné à un instrument ophtalmologique (120) selon la revendication 1, ledit procédé comprenant les étapes suivantes :
a) envoi d'un faisceau image de stimulation (14) pour une valeur de proximité P ;
b) séparation spatiale du faisceau image de stimulation (14) en un faisceau de stimulation oculaire droit (14b) et un faisceau de stimulation oculaire gauche (14a) de manière à stimuler simultanément une accommodation de l'œil droit (13) et de l'œil gauche (12) du sujet ;
c) génération d'un faisceau d'éclairage monoculaire droit (15b) et d'un faisceau d'éclairage monoculaire gauche (15a) dans lequel l'étape c) de génération d'un faisceau d'éclairage monoculaire droit (15b) et d'un faisceau d'éclairage monoculaire gauche (15a) est réalisée en divisant un faisceau d'éclairage binoculaire (15), de manière à éclairer simultanément l'œil droit (13) et l'œil gauche (12) du sujet ;
d) superposition optique dudit faisceau de stimulation oculaire droit (14b) et dudit faisceau d'éclairage monoculaire droit (15b) sur une voie optique droite (17) et respectivement superposition optique dudit faisceau de stimulation oculaire gauche (14a) et dudit faisceau d'éclairage monoculaire gauche (15a) sur une voie optique gauche (16) ;
e) collection séquentielle via le commutateur optique (5) du faisceau de mesure monoculaire droit (55b) ou respectivement du faisceau de mesure monoculaire gauche (55a) suivant un chemin optique séparé du faisceau d'éclairage monoculaire droit (15b), respectivement gauche (15a) ;
f) analyse du faisceau de mesure monoculaire droit (55b) ou respectivement du faisceau de mesure monoculaire gauche (55a), de manière à déterminer au moins un paramètre de vision monoculaire droit, ou respectivement gauche,
g) répétition des étapes c) à f) en commutant le faisceau de mesure monoculaire (55a, 55b) de manière à déterminer au moins un paramètre de vision binoculaire d'un sujet pour une même stimulation oculaire de l'œil droit (13) et de l'œil gauche (12) du sujet.

13. Procédé de détermination d'au moins un paramètre de vision binoculaire objective d'un sujet, comprenant les étapes du procédé selon la revendication 12 pour une première valeur de proximité P1, puis les étapes du procédé selon la revendication 16 pour au moins une autre valeur de proximité Pn.

## Patentansprüche

1. Binokulare Multiplexvorrichtung, kombiniert mit einem ophthalmologischen Instrument (120) mit mehreren Wegen zur objektiven Messung wenigstens eines Sehparameters eines Subjekts, wobei das ophthalmologische Instrument ein Beleuchtungsmittel (200), das geeignet ist, einen Strahl zur binokularen Beleuchtung (15) auszusenden, Mittel zum Erfassen eines durch Reflexion und/oder Refraktion des Beleuchtungsstrahls an einem Auge des Subjekts erzeugten Messstrahls (55) und einen einem einzigen Messweg zugeordneten Sensor (2) aufweist, wobei das Beleuchtungsmittel (200) von dem Sensor (2) getrennt ist,
wobei die binokulare Multiplexvorrichtung umfasst:
- erste Mittel zur optischen Trennung (4), die geeignet sind, einen Stimulationsbild-Strahl (14) zu empfangen, der von einem Stimulations-Sehzeichen (1) ausgeht und dazu bestimmt ist, eine Akkommodation des Subjekts zu stimulieren, wobei die ersten Mittel zur optischen Trennung (4) geeignet sind, den Stimulationsbild-Strahl (14) räumlich in einen Strahl zur Stimulation des rechten Auges (14b) und einen Strahl zur Stimulation des linken Auges (14a) zu trennen, um gleichzeitig eine Akkommodation des rechten Auges (13) und des linken Auges (12) des Subjekts zu stimulieren;
- zweite Mittel zur optischen Trennung (140), die geeignet sind, den Strahl zur binokularen Beleuchtung (15) zu empfangen und den Strahl zur binokularen Beleuchtung (15) in einen Strahl zur rechten monokularen Beleuchtung (15b) und einen Strahl zur linken monokularen Beleuchtung (15a) zu trennen, wobei der Strahl zur rechten monokularen Beleuchtung (15b) bzw. der Strahl zur linken monokularen Beleuchtung (15a) dazu bestimmt sind, gleichzeitig das rechte Auge (13) bzw. das linke Auge (12) des Subjekts zu beleuchten, um nach Reflexion und/oder Refraktion durch das betreffende Auge (12, 13) einen rechten (55b) bzw. linken (55a) okularen Messstrahl zu bilden,
- einen optischen Kommutator (5), der außerhalb des Strahlenganges des Strahls zur Stimulation des rechten Auges (14b), des Strahls zur Stimulation des linken Auges (14a), des Strahls zur rechten monokularen Beleuchtung (15b) und des Strahls zur linken monokularen Beleuchtung (15a) angeordnet ist, wobei der optische Kommutator (5) geeignet ist, den rechten okularen Messstrahl (55b) bzw. den linken okularen Messstrahl (55a) zu empfangen und nacheinander den rechten okularen Messstrahl (55b) bzw. den linken okularen Messstrahl (55a) zu dem Sensor (2) zu leiten, der dem einzigen Messweg zugeordnet ist, wenn der Strahl zur rechten monokularen Beleuchtung (15b) und der Strahl zur linken monokularen Beleuchtung (15a) gleichzeitig das rechte (13) und das linke Auge (12) des Subjekts beleuchten,
- erste Mittel zur optischen Kombination (7), die geeignet sind, den Strahl zur Stimulation des rechten Auges (14b) und den Strahl zur rechten monokularen Beleuchtung (15b) zu überlagern, und erste optische Mittel, um den Strahl zur Stimulation des rechten Auges (14b) und den Strahl zur rechten monokularen Beleuchtung (15b) zum rechten Auge (13) zu lenken, und
- zweite Mittel zur optischen Kombination (6), die geeignet sind, den Strahl zur Stimulation des linken Auges (14a) und den Strahl zur linken monokularen Beleuchtung (15a) zu überlagern, und zweite optische Mittel, um den Strahl zur Stimulation des linken Auges (14a) und den Strahl zur linken monokularen Beleuchtung (15a) zum linken Auge (12) zu lenken.

2. Mit einem ophthalmologischen Instrument kombinierte binokulare Multiplexvorrichtung nach Anspruch 1, wobei die ersten Mittel zur optischen Trennung (4) eine Strahlteilerplatte oder einen Strahlteilerwürfel umfassen.

3. Mit einem ophthalmologischen Instrument kombinierte binokulare Multiplexvorrichtung nach Anspruch 1 oder Anspruch 2, wobei der optische Kommutator (5) eine dichroitische Platte, einen ausrichtbaren Spiegel oder einen wegschwenkbaren Spiegel umfasst.

4. Mit einem ophthalmologischen Instrument kombinierte binokulare Multiplexvorrichtung nach einem der Ansprüche 1 bis 3, wobei die zweiten Mittel zur optischen Trennung eine Strahlteilerplatte oder einen Strahlteilerwürfel umfassen.

5. Mit einem ophthalmologischen Instrument kombinierte binokulare Multiplexvorrichtung nach einem der Ansprüche 1 bis 4, wobei die ersten Mittel zur optischen Kombination (7) eine dichroitische Platte umfassen, die geeignet ist, einen Strahl zur Stimulation des rechten Auges (14b) im sichtbaren Bereich und einen Strahl zur rechten monokularen Beleuchtung (15b) im Infrarotbereich zu überlagern, und/oder die zweiten Mittel (6) zur optischen Kombination eine dichroitische Platte umfassen, die geeignet ist, einen Strahl zur Stimulation des linken Auges (14a) im sichtbaren Bereich und einen Strahl zur linken monokularen Beleuchtung (15a) im Infrarotbereich zu überlagern.

6. Mit einem ophthalmologischen Instrument kombinierte binokulare Multiplexvorrichtung nach einem der Ansprüche 1 bis 5, welche eine rechte Austrittspupille (37), eine linke Austrittspupille (36) und optische Bildgebungsmittel (20, 21, 23, 32, 33, 34) umfasst, wobei die rechte Austrittspupille (37) geeignet ist, den Strahl zur rechten monokularen Beleuchtung (15b) und den rechten okularen Messstrahl (55b) zu empfangen, bzw. die linke Austrittspupille (36) geeignet ist, den Strahl zur linken monokularen Beleuchtung (15a) und den linken okularen Messstrahl (55a) zu empfangen, und die optischen Bildgebungsmittel (18, 19, 20, 21, 23, 32, 33, 34) geeignet sind, das Bild einer Austrittspupille (22, 35) des ophthalmologischen Instruments mit einem einzigen Messweg auf der rechten Austrittspupille (37) und/oder auf der linken Austrittspupille (36) zu bilden.

7. Mit einem ophthalmologischen Instrument kombinierte Multiplexvorrichtung nach Anspruch 6, welche Mittel zur sekundären Beleuchtung (160, 161) umfasst, die geeignet sind, einen Strahl zur sekundären Beleuchtung (170, 171) in Richtung der Hornhaut jedes Auges (12, 13) auszusenden, um einen rechten (55b) bzw. linken (55a) okularen Messstrahl zu erzeugen, um eine Analyse der Hornhaut durch Keratometrie und/oder Hornhauttopographie zu liefern.

8. Mit einem ophthalmologischen Instrument kombinierte binokulare Multiplexvorrichtung nach einem der Ansprüche 1 bis 7, welche außerdem ein im Strahlengang des Stimulationsbild-Strahls (14) angeordnetes optisches System (3) umfasst, wobei das optische System (3) geeignet ist, einen mittleren Sphärenfehler der beiden Augen (12, 13) auszugleichen.

9. Mit einem ophthalmologischen Instrument kombinierte binokulare Multiplexvorrichtung nach einem der Ansprüche 1 bis 8, welche außerdem ein im Strahlengang des Strahls zur Stimulation des rechten Auges (14b) angeordnetes erstes optisches System (18, 26) und ein im Strahlengang des Strahls zur Stimulation des linken Auges (14a) angeordnetes zweites optisches System (19, 27) umfasst, wobei das erste bzw. zweite optische System (18, 26, 19, 27) geeignet ist, einen Sphären- und/oder Zylinderfehler des rechten (13) bzw. linken Auges (12) des Subjekts auszugleichen.

10. Mit einem ophthalmologischen Instrument kombinierte binokulare Multiplexvorrichtung nach einem der Ansprüche 1 bis 9, welche umfasst:
- ein rechtes optisches System (9, 11, 21), das zwischen den ersten Mitteln zur optischen Kombination (7) und dem rechten Auge (13) des Subjekts angeordnet ist,
- ein linkes optisches System (8, 10, 20), das zwischen den zweiten Mitteln zur optischen Kombination (6) und dem linken Auge (12) des Subjekts angeordnet ist, und
- Mittel zur Ausrichtung (30, 31, 32, 33) des rechten optischen Systems (9, 11, 21, 33) und des linken optischen Systems (8, 10, 20, 32), um die Pupillendistanz in Abhängigkeit von der prismatischen Konvergenz des Subjekts anzupassen.

11. Mit einem ophthalmologischen Instrument kombinierte binokulare Multiplexvorrichtung nach einem der Ansprüche 1 bis 10, welche eine elektronische Verarbeitungseinheit umfasst, die dafür ausgelegt ist, ein Signal zu empfangen, das für den Kommutationszustand des optischen Kommutators (5) repräsentativ ist, und nacheinander einen ersten Messwert, der für den rechten okularen Messstrahl (55b) repräsentativ ist, und einen zweiten Messwert, der für den linken okularen Messstrahl (55a) repräsentativ ist, zu empfangen, wobei die elektronische Verarbeitungseinheit geeignet ist, das Signal, das für den Kommutationszustand repräsentativ ist, und den ersten und den zweiten Messwert zu kombinieren, um einen gemultiplexten binokularen Messwert wenigstens eines Sehparameters zu liefern.

12. Verfahren zur Bestimmung wenigstens eines binokularen Sehparameters eines Subjekts, welches auf der Verwendung einer mit einem ophthalmologischen Instrument (120) kombinierten binokularen Multiplexvorrichtung nach Anspruch 1 basiert, wobei das Verfahren die folgenden Schritte umfasst:
a) Senden eines Stimulationsbild-Strahls (14) für einen Nahbereichswert P;
b) räumliche Trennung des Stimulationsbild-Strahls (14) in einen Strahl zur Stimulation des rechten Auges (14b) und einen Strahl zur Stimulation des linken Auges (14a), um gleichzeitig eine Akkommodation des rechten Auges (13) und des linken Auges (12) des Subjekts zu stimulieren;
c) Erzeugung eines Strahls zur rechten monokularen Beleuchtung (15b) und eines Strahls zur linken monokularen Beleuchtung (15a), wobei der Schritt c) der Erzeugung eines Strahls zur rechten monokularen Beleuchtung (15b) und eines Strahls zur linken monokularen Beleuchtung (15a) ausgeführt wird, indem ein Strahl zur binokularen Beleuchtung (15) geteilt wird, um gleichzeitig das rechte Auge (13) und das linke Auge (12) des Subjekts zu beleuchten;
d) optische Überlagerung des Strahls zur Stimulation des rechten Auges (14b) und des Strahls zur rechten monokularen Beleuchtung (15b) auf einem rechten optischen Weg (17) und optische Überlagerung des Strahls zur Stimulation des linken Auges (14a) und des Strahls zur linken monokularen Beleuchtung (15a) auf einem linken optischen Weg (16);
e) nacheinander erfolgende Erfassung, über den optischen Kommutator (5), des rechten monokularen Messstrahls (55b) bzw. des linken monokularen Messstrahls (55a) entlang eines getrennten Strahlenganges des Strahls zur rechten (15b) bzw. linken (15a) monokularen Beleuchtung;
f) Analyse des rechten monokularen Messstrahls (55b) bzw. des linken monokularen Messstrahls (55a), um wenigstens einen rechten bzw. linken monokularen Sehparameter zu bestimmen;
g) Wiederholung der Schritte c) bis f), dabei Kommutieren des monokularen Messstrahls (55a, 55b), um wenigstens einen binokularen Sehparameter eines Subjekts für dieselbe Augenstimulation des rechten Auges (13) und des linken Auges (12) des Subjekts zu bestimmen.

13. Verfahren zur Bestimmung wenigstens eines objektiven binokularen Sehparameters eines Subjekts, welches die Schritte des Verfahrens nach Anspruch 12 für einen ersten Nahbereichswert P1 und anschließend die Schritte des Verfahrens nach Anspruch 16 für wenigstens einen weiteren Nahbereichswert Pn umfasst.

## Claims

1. A binocular multiplexing device combined to an ophthalmological instrument (120) having several objective measuring channels of at least one visual parameter of a subject, said ophthalmological instrument comprising an illumination mean (200) able to emit a binocular illuminating beam (15), means for collecting a measuring beam (55) generated by reflection and/or refraction of said illuminating beam from an eye of the subject, and a sensor (2) associated with a single measuring channel, the illumination mean (200) being separated from said sensor (2), said binocular multiplexing device comprising:
- first optical splitting means (4) able to receive a stimulating image beam (14) issued from a stimulus test pattern and intended to stimulate accommodation by the subject, said first optical splitting means (4) being able to split said stimulating image beam (14) into a right ocular stimulating beam (14b) and a left ocular stimulating beam (14a) so as to stimulate accommodation by the right eye (13) and left eye (12) of the subject simultaneously ;
- second optical splitting means (140) able to receive said binocular illuminating beam (15) and to split said binocular illuminating beam (15) into a right monocular illuminating beam (15b) and a left monocular illuminating beam (15a), said right monocular illuminating beam (15b) and respectively said left monocular illuminating beam (15a) being intended to illuminate the right eye (13) and the left eye (12) of the subject, respectively, in order to form, after reflection and/or refraction from the eye (12, 13) in question, a right ocular measuring beam (55b), respectively a left ocular measuring beam (55a);
- an optical switch (5) placed outside the optical path of the right ocular stimulating beam (14b), left ocular stimulating beam (14a), right monocular illuminating beam (15b) and left monocular illuminating beam (15a), said optical switch being able to receive said right ocular measuring beam (55b) and respectively said left ocular measuring beam (55a) and to steer in sequence said right ocular measuring beam (55b) or respectively said left ocular measuring beam (55a), toward said sensor (2) associated with said single measuring channel, when the right monocular illuminating beam (15b) and respectively the left monocular illuminating beam (15a) illuminate simultaneously the right eye (13) and the left eye (12) of the subject;
- first optical combining means (7) able to superpose the right ocular stimulating beam (14b) and the right monocular illuminating beam (15b), and first optical means for directing the right ocular stimulating beam (14b) and the right monocular illuminating beam (15b) toward the right eye (13); and
- second optical combining means (6) able to superpose the left ocular stimulating beam (14a) and the left monocular illuminating beam (15a), and second optical means for directing the left ocular stimulating beam (14a) and the left monocular illuminating beam (15a) toward the left eye (12).

2. The binocular multiplexing device combined to an ophthalmological instrument as claimed in claim 1, in which said first optical splitting means (4) comprise a beam-splitting plate or a beam-splitting cube.

3. The binocular multiplexing device combined to an ophthalmological instrument as claimed in claim 1 or in claim 2, in which said optical switch (5) comprises a dichroic plate, an orientable mirror, a retractable mirror.

4. The binocular multiplexing device combined to an ophthalmological instrument as claimed in one of claims 1 to 3, in which said second optical splitting means comprise a beam-splitting plate or a beam-splitting cube.

5. The binocular multiplexing device combined to an ophthalmological instrument as claimed in one of claims 1 to 4, in which said first optical combining means (7) comprise a dichroic plate able to superpose a right ocular stimulating beam (14b) in the visible domain and a right monocular illuminating beam (15b) in the infrared domain, and/or respectively said second optical combining means (6) comprise a dichroic plate able to superpose a left ocular stimulating beam (14a) in the visible domain and a left monocular illuminating beam (15a) in the infrared domain.

6. The binocular multiplexing device combined to an ophthalmological instrument as claimed in one of claims 1 to 5, comprising a right exit pupil (37), a left exit pupil (36) and imaging optical means (20, 21, 23, 32, 33, 34), said right exit pupil (37) being able to receive said right monocular illuminating beam (15b) and said right ocular measuring beam (55b), respectively said left exit pupil (36) being able to receive said left monocular illuminating beam (15a) and said left ocular measuring beam (55a), , and said imaging optical means (18, 19, 20, 21, 23, 32, 33, 34) being able to form the image of an exit pupil (22, 35) of said ophthalmological instrument having a single measuring channel on said right exit pupil (37) and/or said left exit pupil (36).

7. The binocular multiplexing device combined to an ophthalmological instrument as claimed in claim 6, comprising secondary illuminating means (160, 161) able to emit a secondary illuminating beam (170, 171) in the direction of the cornea of each eye (12, 13) so as to generate a right ocular measuring beam (55b) and respectively a left ocular measuring beam (55a), in order to provide an analysis of the cornea by keratometry and/or corneal topography.

8. The binocular multiplexing device combined to an ophthalmological instrument as claimed in one of claims 1 to 7, furthermore comprising an optical system (3) placed on the optical path of the stimulating image beam (14), said optical system (3) being able to correct an average sphere error of the two eyes (12, 13).

9. The binocular multiplexing device combined to an ophthalmological instrument as claimed in one of claims 1 to 8, furthermore comprising a first optical system (18, 26) placed on the optical path of the right ocular stimulating beam (14b) and a second optical system (19, 27) placed on the optical path of the left ocular stimulating beam (14a), said first optical system (18, 26), respectively said second optical system (19, 27) being able to correct a sphere and/or cylinder error of the right eye (13), respectively of the left eye (12), of the subject.

10. The binocular multiplexing device combined to an ophthalmological instrument as claimed in one of claims 1 to 9, comprising:
- a right optical system (9, 11, 21) placed between the first optical combining means (7) and the right eye (13) of the subject;
- a left optical system (8, 10, 20) placed between the second optical combining means (6) and the left eye (12) of the subject; and
- means (30, 31, 32, 33) for aligning said right optical system (9, 11, 21, 33) and said left optical system (8, 10, 20, 32) so as to adjust the pupillary distance depending on the prismatic convergence of the subject.

11. The binocular multiplexing device combined to an ophthalmological instrument as claimed in one of claims 1 to 10 comprising an electronic processing unit suitable for receiving a signal representative of the switching state of said optical switch (5) and to receive, in sequence, a first measurement representative of said right ocular measuring beam (55b) and a second measurement representative of said left ocular measuring beam (55a), said electronic processing unit being able to combine said signal representative of the switching state and said first and second measurements in order to deliver a multiplexed binocular measurement of at least one vision parameter.

12. A method for determining at least one binocular vision parameter of a subject, based on the use of a binocular multiplexing device combined to an ophthalmological instrument (120) as claimed in claim 1, said method comprising the following steps:
a) delivering a stimulating image beam (14) for a proximity value P;
b) splitting the stimulating image beam (14) into a right ocular stimulating beam (14b) and a left ocular stimulating beam (14a) so as to stimulate accommodation by the right eye (13) and by the left eye (12) of the subject simultaneously;
c) generating a right monocular illuminating beam (15b) and a left monocular illuminating beam (15a), wherein said step c) of generating a right monocular illuminating beam (15b) and a left monocular illuminating beam (15a) is made by splitting a binocular illuminating beam (15), so as to illuminate simultaneously the right eye (13) and the left eye (12) of the subject;
d) optically superposing said right ocular stimulating beam (14b) and said right monocular illuminating beam (15b) in a right optical channel (17) and respectively optically superposing said left ocular stimulating beam (14a) and said left monocular illuminating beam (15a) in a left optical channel (16);
e) collecting in sequence via the optical switch (5) the right monocular measuring beam (55b) or respectively the left monocular measuring beam (55a) along an optical path that is the inverse of that of the right monocular illuminating beam (15b) and respectively of the left monocular illuminating beam (15a);
f) analyzing the right monocular measuring beam (55b) or respectively the left monocular measuring beam (55a) so as to determine at least one right monocular vision parameter or respectively left monocular vision parameter; and
g) repeating steps c) to f) after switching of the monocular measuring beam (55a, 55b), so as to determine at least one binocular vision parameter of a subject using the same ocular stimulations for the right eye (13) and left eye (12) of the subject.

13. A method for determining at least one objective binocular vision parameter of a subject, comprising carrying out the steps of the method as claimed in claim 12 for a first proximity value P1, then carrying out the steps of the method according to claim 16 for at least one other proximity value Pn.
